Europäisches Patentamt

European Patent Office

Office européen des brevets

(19)

(11) Publication number: **0 253 415**
**B1**

# EUROPEAN PATENT SPECIFICATION

(12)

(45) Date of publication of the patent specification:
14.03.90

(51) Int. Cl.⁴: **C07J 1/00**

(21) Application number: **87201114.3**

(22) Date of filing: **12.06.87**

(54) **Process for the preparation of 9(11)-dehydroandrostanes.**

(30) Priority: **07.07.86 NL 8601790**

(43) Date of publication of application:
**20.01.88 Bulletin 88/3**

(45) Publication of the grant of the patent:
**14.03.90 Bulletin 90/11**

(84) Designated Contracting States:
**AT BE CH DE ES FR GB GR IT LI LU NL SE**

(56) References cited:
**DD-A- 20 528**
**FR-A- 2 387 245**
**US-A- 4 102 907**

(73) Proprietor: **GIST-BROCADES N.V., Wateringseweg 1,
NL-2611 XT Delft(NL)**

(72) Inventor: **Batist, Jacobus Nicolaas M., Lange
Waateringkade 33a, NL-2295 RN Kwintsheul(NL)**
Inventor: **Marx, Arthur Friedrich, Florence
Nightingalelaan 12, NL-2614 GM Delft(NL)**

(74) Representative: **Huygens, Arthur Victor, Dr. et al, c/o
Gist-Brocades N.V. Patent & Trademarks Department
Wateringseweg 1 PO Box 1, NL-2600 MA Delft(NL)**

## Description

This invention relates to the preparation of 9(11)-dehydroandrostanes from the corresponding 9-alphahydroxy compounds by dehydration.

The 9(11)-dehydroandrostanes constitute an important group of intermediates in the synthesis of steroid drugs. They can easily be transformed into steroids with a 9-halogen and/or an 11-hydroxy substituent, which substituents are characteristic of a large number of compounds belonging to the group of corticosteroids.

Various processes for the preparation of 9(11)-dehydrosteroids are already known, e.g. by dehydration of the corresponding 9-alpha-hydroxy compounds.

US patent 3 065 146 describes dehydration using thionyl chloride in pyridin for the preparation of 9(11)-dehydroprogesterone. This method is also used for the dehydration of 9-alpha-hydroxyandrost-4-ene-3,17-dione.

Dutch patent application NL 7 802 302 indicates that with the above procedure also a considerable amount of the 8(9)-dehydro isomer is produced. This cause a lowering of the yield of the desired compound and also has a further disadvantage in that the removal of the unwanted isomer is very complicated and costly.

East German patent DD 20 528 provides an easy method of preparation of 9(11)-dehydrosteroids, comprising boiling a solution of the corresponding 9-alpha-hydroxy compound with an aromatic sulphonic acid, especially p-toluenesulphonic acid. Although the use of 9-alpha-hydroxytestosterone is specifically mentioned, the examples only refer to pregnanes. There is hardly any data given in the patent specification referring to yield and purity.

The method fails when applied to 9-alpha-hydroxyandrostanes. No 9(11)-dehydroandrostane is formed at all which is confirmed by C.G. Bergstrom and R.M. Dodson (Chemistry and Industry, 1530 (1961)).

An improved process is descirbed in German patent application DE 2 806 687. The 9-alpha-hydroxyl group of an androstane compound is converted first into a 9-alpha-OSOR-group (R is (1–4C)alkyl, phenyl or substituted phenyl) by reaction with a sulfinylchloride. By boiling the product subsequently in benzene with silica gel or alumina and an acid, a desulfination reaction takes place, giving the desired 9(11)-dehydro compound in yields of at least 85% and with a ratio 9(11)-dehydro:8(9)-dehydro isomers being at least 98:2. However, in spite of the good yeils of 9(11)-isomer the additional step constitutes a practical and an economical disadvantage.

The fact that the art believed p-toluenesulfonic acid to be unsuitable to dehydrate 9-alpha-hydroxyandrostanes is perhaps the reason why German patent application DE 2 814 747 did not refer explicitly to the use of aromatic sulfonic acids. With nonaromatic, oxygen containing acids good results were obtained.

The present invention provides a method for the preparation of 9(11)-dehydroandrostanes in a high yield by dehydrating the corresponding 9-alpha-hydroxyandrostanes, with the undesired 8(9)-dehydro isomer not being present in significant amounts.

According to the invention, the dehydration is carried out in the presence of an aromatic sulfonic acid and silica gel. Preferably the aromatic sulfonic acid is p-toluenesulfonic acid or naphtalenesulfonic acid, which are cheap and readily available reagents.

The silica gel may be added to the solution either admixed with the acid, or separately or as an adsorbate of the aromatic sulfonic acid on silica gel. An example of the preparation of an adsorbate of an aromatic sulfonic acid on silica gel is described in Synthesis 1985, pp. 1159–1161. In this reference a p-toluenesulphonic acid adsorbate appears to be used for the dehydration of 3-hydroxysteroids yielding 2(3)- or 3(4)-dehydro steroids.

Suitable conditions for the dehydration are: boiling of the 9-alpha-hydroxyandrostane in an organic solvent, e.g. benzene, chlorobenzene and preferably toluene, for a period which may vary from several minutes to several hours, preferably from 5 minutes to three hours. The dehydration reaction may be monitored using thin layer chromatography. The reaction temperature preferably is at least 80°C.

A great advantage of the process according to the invention is that as previously mentioned the resulting 9(11)-dehydroandrostane contains hardly any or none amount of the troublesome 8(9)-dehydroisomer. This isomer may be not distinguishable from the 9(11)-isomer by thin layer chromatography because the Rf-values hardly differ. But sensitive 360 MHz NMR-equipment can detect an amount as littly as 0.5% of 8(9)-dehydro steroid using a difference of 12 Hz between C(4)H-shifts of both isomers. In the product prepared according to the invention there was no indication of the 8(9)-dehydro isomer when tested by this method.

Therefore the present invention provides a cheap and easy method for the preparation in high yield and in a high grade of purity of an important group of 9(11)-dehydro steroids.

The following examples figure as illustrations of the invention.

### Example 1

#### Adsorbate of p-toluenesulfonic acid on silica gel

A solution of 3 g of p-toluenesulfonic acid monohydrate, or 2-naphtalene-sulfonic acid in 20 ml of acetone is rapidly added to silica gel under vigorous stirring.

After 1 hour the solvent is removed under reduced pressure at 40–50°C for 1 night yielding the adsorbate.

Examples 2–14 present preparations of androsta-4,9(11)-diene-3,17-dione by dehydration of the corresponding 9-alpha-hydroxy compound using several acid catalysts. In Example 15 no silica gel is used. Yields are established using High Performance Liquid Chromatography (HPLC).

## Example 2

Following Example 1 an adsorbate was made of about 0.1 g of p-toluenesulfonic acid monohydrate and 3 g of silica gel (Merck 60, particle size 0.063–0.200 mm, 70–230 mesh). This adsorbate is mixed under vigorous stirring, with a solution of 0.18 g of 9-alpha-hydroxyandrost-4-ene-3,17-dione in 40 ml of dry benzene. Stirring is continued for two hours at reflux temperature. Subsequently the mixture is transferred into an empty chromatography column and thereupon eluted with a 1:1 mixture of toluene and acetone. The eluate was evaporated to dryness, yielding 0.22 g crude androsta-4,9(11)-diene-3,17-dione.

NMR (360 MHz; CDCl$_3$; TMS): $\delta$ 0.891 (C(18)H$_3$, s, 3H), 1.372 (C(19)H$_3$, s, 3H), 5.57 (C(11)H, m, 1H), 5.76 (C(4)H, s, 1H). Androsta-4,8-diene-3,17-dione could not be detected. The product was purified by crystallisation from an acetone/water mixture.

## Example 3

The process of Example 2 was repeated, except that silica gel (1.5 g) and p-toluenesulfonic acid monohydrate (0.1 g) were added separately. A similar result to that of Example 2 was obtained.

## Example 4

A suspension of 2.5 g of 9-alpha-hydroxyandrost-4-ene-3,17-dione and 30 g of an adsorbate of 2-naphtalene-sulfonic acid on chromatographic grade silica gel (Merck 60, particle size 0.063–0.2 mm) in toluene was stirred for 30 minutes at reflux temperature. Then the mixture was transferred into an empty chromatography column and thereupon eluted with 250 ml of acetone. The eluate was evaporated to dryness and analyzed by HPLC. According to HPLC-analysis 60.8% of androsta-4,9(11)-diene-3,17-dione was formed.

## Example 5

The process of Example 4 was repeated, except that instead of 2-naphtalenesulfonic acid p-toluenesulfonic acid monohydrate was used. Yield 53.1%.

## Example 6

The process of Example 4 was repeated except that an absorbate of 2-naphtalenesulfonic acid on TLC-grade slilica gel (Merck 60H, particle size 0.005–0.040 mm) was used. Stirring for 10 minutes resulted in a yield of 45.6%, stirring for 30 minutes resulted in a yield of 65.8%.

## Example 7

The process of Example 6 was repeated, except that instead of 2-naphtalenesulfonic acid p-toluenesulfonic acid monohydrate was used. According to HPLC-analysis stirring for 30 and 60 minutes resulted in 57.9 and 62.5% yield, respectively.

## Example 8

The process of Example 4 was repeated except that instead of toluene benzene was used. According to HPLC 43% of androsta-4,9(11)-diene-3,17-dione was formed.

## Example 9

The process of Example 4 was repeated except that silica gel of the types Merck Lichoprep. Si 60 (0.005–0.020 mm) resp. Merck Lichoprep. Si 60 (0.015–0.0025 mm) was used. Yields were 38.8 and 46.9%, respectively.

## Example 10

To a stirring solution of 2.5 g of 9-alpha-hydroxyandrost-4-ene-3,17-dione in 300 ml of toluene 0.9 g 2-naphtalenesulfonic acid and 30 g of chromatographic grade silica gel (Merck 60, particle size 0.063–0.200 mm, dried for 90 hrs at 150°C) were added separately. The suspension was stirred for 30 minutes at reflux temperature. Subsequently the mixture was transferred into an empty chromatography column and thereupon eluted with 250 ml of acetone. The eluate was evaporated to dryness and analyzed by HPLC.

Yield: 49.1% of androsta-4,9(11)-diene-3,17-dione.

## Example 11

The process of Example 10 was repeated, except that an adsorbate of 2-naphtalenesulfonic acid on dry silica gel was used. Yield 45.6%.

## Example 12

The process of Example 4 was repeated except that an adsorbate of p-toluenesulfonic acid monohydrate on TLC-grade silica gel (Merck 60H) was used. Stirring for 30 minutes at 60°C, 80°C, 100°C and reflux temperature resulted in yields of 2.3%, 28.9%, 53.3% and 57.9%, respectively.

## Example 13

The process of example 7 was repeated except that instead of toluene chlorobenzene was used. Stirring for 30 minutes at reflux temperature resulted in a yield of 60.3%.

## Example 14

The process of Example 10 was repeated except that TLC-grade silica gel (Merck 60 H) was used and instead of toluene chlorobenzene was used. Yield 43.4%.

## Example 15

A solution of 2.5 g of 9-alpha-hydroxyandrost-4-ene-3,17-dione and 0.9 g of 2-naphtalenesulfonic acid in 300 ml toluene was refluxed for 30 minutes without any silica gel. The reaction mixture was evaporated to dryness and analyzed by HPLC. According to HPLC no androsta-4,9(11)-diene-3,17-dione could be detected. The remaining amount of starting material was 11%.

## Claims

1. Process for the preparation of a 9(11)-dehydroandrostane by dehydration of the corresponding 9-alpha-hydroxyandrostane, characterized in that the dehydration is carried out in the presence of an aromatic sulfonic acid and silica gel.

2. Process according to claim 1, characterized in that the dehydration is carried out by heating a solution of the steroid in the presence of an aromatic sulfonic acid and silica gel.

3. Process according to claim 1 or 2, characterized in that the aromatic sulfonic acid is p-toluenesulfonic acid or naphtalenesulfonic acid.

## Patentansprüche

1. Verfahren zur Herstellung eines 9(11)-Dehydroandrostans durch Dehydratisierung des entsprechenden 9-alpha-Hydroxyandrostans, dadurch gekennzeichnet, dass die Dehydratisierung in Gegenwart einer aromatischen Sulfonsäure und von Kieselgel ausgeführt wird.

2. Verfahren nach Anspruch 1, dadurch gekennzeichnet, dass die Dehydratisierung ausgeführt wird, indem man eine Lösung des Steroides in Gegenwart einer aromatischen Sulfonsäure und von Kieselgel erhitzt.

3. Verfahren nach Anspruch 1 oder 2, dadurch gekennzeichnet, dass die aromatische Sulfonsäure p-Toluolsulfonsäure oder Naphthalinsulfonsäure ist.

## Revendications

1. Procédé pour la préparation d'un 9(11)-déshydro-androstane par déshydratation du 9-alpha-hydroxy-androstane correspondant, caractérisé en ce que la déshydratation est exécutée en présence d'un acide sulfonique aromatique et de gel de silice.

2. Procédé suivant la revendication 1, caractérisé en ce que la déshydratation est exécutée par chauffage d'une solution du stéroïde en présence d'un acide sulfonique aromatique et de gel de silice.

3. Procédé suivant la revendication 1 ou 2, caractérisé en ce que l'acide sulfonique aromatique est l'acide p-toluènesulfonique ou l'acide naphtalènesulfonique.